# EUROPÄISCHE PATENTSCHRIFT

(11) **EP 1 646 334 B1**
(45) Veröffentlichungstag und Bekanntmachung des Hinweises auf die Patenterteilung: **10.09.2008**
(21) Anmeldenummer: 03739936.7
(22) Anmeldetag: 22.07.2003
(51) Int. Cl.: A61F 2/30, A61F 2/44

(54) **GELENKENDOPROTHESE**
ARTICULATED ENDOPROSTHESIS
ENDOPROTHESE D'ARTICULATION

(43) Veröffentlichungstag der Anmeldung: 19.04.2006
(73) Patentinhaber: Synthes GmbH, 4436 Oberdorf (CH)
(72) Erfinder: LECHMANN, Beat, CH-2544 Bettlach (CH); BÜRKI, Roger, CH-4710 Balsthal (CH); BURKARD, Dominique, CH-5014 Gretzenbach (CH); FRIGG, Robert, CH-2544 Bettlach (CH); ODERMATT, Daniel, CH-4500 Solothurn (CH)
(74) Vertreter: Lusuardi, Werther
(86) Internationale Anmeldenummer: PCT/CH2003/000494
(87) Internationale Veröffentlichungsnummer: WO 2005/007038

(56) Entgegenhaltungen:
- DE-A- 10 037 504
- US-A- 3 658 056
- US-A- 5 662 158
- US-A- 5 879 407

## Beschreibung

Die Erfindung bezieht sich auf eine Gelenkendoprothese, insbesondere auf ein Zwischenwirbelimplantat gemäss dem Oberbegriff des Patentanspruchs 1.

Die meisten der gegenwärtig eingesetzten Zwischenwirbelimplantate oder Bandscheibenprothesen umfassen ein Gelenk, dessen Gelenkflächen aus Metall, Polymeren oder keramischen Werkstoffen bestehen. Die Gelenkflächen bleiben üblicherweise trocken, wodurch die Lebensdauer des Implantates beeinträchtigt werden kann. Zudem sind wegen der begrenzten Rotationsbewegungen der auf einander gleitbar gelagerten Gelenkteile die tribologischen Eigenschaften relevant.

Aus der EP-B 0 193 538 ist eine künstliche Hüftpfanne bekannt, welche mit einer Flüssigkeitskammer ausgestattet ist, um die tribologischen Eigenschaften der Gelenkflächen zu verbessern. Nachteilig bei diesem Implantat ist der Umstand, dass die Flüssigkeitskammer geschlossen ist, so dass keine Körperflüssigkeit von Aussen angesogen werden kann, sondern nur aus der Kammer selbst. Ferner kann sich in geschlossenen Flüssigkeitskammern Körperflüssigkeit ansammeln, welche durch dünne Ritzen von aussen in die Flüssigkeitskammer eindringen kann. Wegen der fehlenden Zirkulation kann sich der pH-Wert dieser angesammelten Flüssigkeit absenken, wodurch die Teile des Implantates chemisch angegriffen werden können. Zudem können die Relativbewegungen der Implantatteile bei geschlossenen Flüssigkeitskammern, welche durch solche Ritzen mit den Aussenflächen des Implantates verbunden sind, einen unerwünschten Pumpeffekt verursachen.

Die US 5,879,407, die US 3,658,056 so wie die DE 100 37 504 offenbaren je eine Femurkomponente, welche mit Kanälen versehen ist.

Hier will die Erfindung Abhilfe schaffen. Der Erfindung liegt die Aufgabe zugrunde, eine Gelenkprothese zu schaffen, welche einen oder mehrere in die Grenzflächen zwischen Implantat und Knochen mündende Kanäle umfasst, die für die Zufuhr von Körperflüssigkeiten, welche Proteine und andere Substanzen mit guten Schmiereigenschaften enthalten, an die aufeinander gleitbar gelagerten Gelenkflächen geeignet sind.

Die Erfindung löst die gestellte Aufgabe mit einer Gelenkendoprothese, welche die Merkmale des Anspruchs 1 aufweist.

Weitere vorteilhafte Ausgestaltungen der Erfindung sind in den abhängigen Ansprüchen gekennzeichnet.

Die durch die Erfindung erreichten Vorteile sind im wesentlichen darin zu sehen, dass dank der erfindungsgemässen Gelenkendoprothese
- Körperflüssigkeit zu den Gelenkflächen transportierbar ist;
- insbesondere bei deren Ausgestaltung als Zwischenwirbelimplantat die Öffnungen der Kanäle zentral in den Appositionsflächen angeordnet sein können, so dass die Zufuhr der Körperflüssigkeiten analog zur natürlichen Bandscheibe erfolgen kann; und
- andere Körperflüssigkeiten durch die Kavität, welche durch die Inzision entsteht, an die Gelenkflächen fliessen können.

In einer bevorzugten Ausführungsform sind die unteren und die oberen Kanäle derart angeordnet, dass mindestens ein oberer Kanal in die obere Appositionsfläche und mindestens ein unterer Kanal in die untere Appositionsfläche mündet, so dass beidseitig Körperflüssigkeit an die Gelenkflächen geführt werden kann.

In einer anderen Ausführungsform dringt mindestens ein unterer oder mindestens ein oberer Kanal von den Seitenflächen des Zwischenwirbelimplantates in das entsprechende Gelenkteil ein, so dass auch von der durch die Inzision geschaffenen Kavität Körperflüssigkeit an die Gelenkflächen transportierbar ist.

Vorzugsweise sind die Mündungen der Kanäle in die Gelenkflächen A;B mit Rundungen versehen. Dadurch ist der Vorteil erreichbar, dass keine Schädigungen an den Gelenkflächen, hervorgerufen durch scharfkantige Übergänge, entstehen.

In wiederum einer anderen Ausführungsform umfasst das Zwischenwirbelimplantat mehrere obere Kanäle und vorzugsweise auch mehrere untere Kanäle, welche derart angeordnet sind, dass ihre Längsachsen mit der Zentralachse des Zwischenwirbelimplantates einen Winkel zwischen 0° und 90° einschliessen. Die Winkel sind so gewählt, dass
- die Festigkeit des Zwischenwirbelimplantates ausreichend bleibt;
- die Kanäle an gewünschten Positionen in die Gelenkflächen münden; und
- die Kanalmündungen aussen dort am Zwischenwirbelimplantat angeordnet sind, wo Gelenkflüssigkeit vorhanden ist, insbesondere zentral in den Appositionsflächen und bei der durch die Inzision geschaffenen Kavität.

In einer weiteren Ausführungsform weist jeder Kanal eine Querschnittsfläche auf, welche zwischen 0,01 % und 10% einer Gelenkfläche A;B beträgt. Geeignete Abmessung hierzu sind im Falle einer als Zwischenwirbelimplantat ausgebildeten Gelenkendoprothese:
- Gelenkfläche A' zwischen 400 mm² und 500 mm²;
- Gelenkfläche A" zwischen 200 mm² und 300 mm²;
- Minimale Querschnittsfläche eines Kanals 0,07 mm² ;
- Maximale Querschnittsfläche eines Kanals 12,6 mm²;
- Appositionsfläche 969 mm² .

Vorzugsweise weisen die Kanäle einen Durchmesser zwischen 0,3 mm und 4,0 mm auf, wobei der minimale Durchmesser auf die Grösse der Partikel abgestimmt ist, welche eine schmierende Eigenschaft aufweisen, beispielsweise Proteine. Der maximale Durchmesser der Kanäle ist so gewählt, dass am Zwischenwirbelimplantat keine signifikante Schwächung der Festigkeit auftritt.

In wiederum einer weiteren Ausführungsform münden die Kanäle symmetrisch verteilt in die Gelenkflächen A;B. Die obere Appositionsfläche weist einen Flächeninhalt Fₒ auf während die oberen Kanäle innerhalb einer Teilfläche mit einem Flächeninhalt Tₒ < Fₒ in die obere Appositionsfläche münden. Vorzugsweise beträgt der Flächeninhalt Tₒ der Teilfläche zwischen 0,006 % und 1,0 % des Flächeninhaltes Fₒ der oberen Appositionsfläche. Analog weist die untere Appositionsfläche einen Flächeninhalt Fᵤ auf, während die unteren Kanäle innerhalb einer Teilfläche mit einem Flächeninhalt Tᵤ < Fᵤ in die untere Appositionsfläche münden, wobei der Flächeninhalt Tᵤ der Teilfläche vorzugsweise zwischen 0,006 % und 1,0 % des Flächeninhaltes Fᵤ der unteren Appositionsfläche beträgt. Ferner sind diese Teilflächen vorzugsweise konzentrisch zur Zentralachse des Zwischenwirbelimplantates angeordnet.

Das Gelenk kann in verschiedenen Ausführungsformen zwei oder drei Gelenkteile umfassen. Als Materialpaarungen für die Gelenkteile sind die folgenden Paarungen besonders geeignet:
- Metall - Polymer
- Metall - Metall
- Keramik - Keramik
wobei als Metalle besonders Legierungen aus Kobalt, Chrom und Molybdän geeignet sind. Bei einer Metallpaarung Metall - Metall kann die Legierung noch mit Kohlenstoff angereichert werden. Ebenso kann die Oberfläche eines aus Metall gefertigten Gelenkteiles mittels eines geeigneten Verfahrens mit Titankarbid, Titannitrid oder amorphem Kohlenstoff beschichtet werden. Bei dem Polymer handelt es sich vorzugsweise um hochmolekulares Polyethylen (UHMWPE), wobei die Oberfläche durch ein geeignetes Verfahren vernetzt werden kann, um die Verschleisseigenschaften zu verbessern.

Die Erfindung und Weiterbildungen der Erfindung werden im folgenden anhand der teilweise schematischen Darstellungen mehrerer Ausführungsbeispiele noch näher erläutert.

Es zeigen:
Fig. 1a einen medio-lateralen Schnitt durch eine Ausführungsform der erfindungsgemässen Gelenkendoprothese;
Fig. 1b einen Ausschnitt aus dem unteren Gelenkteil der in Fig. 1a dargestellten Ausführungsform der erfindungsgemässen Gelenkendoprothese mit abgerundeten Mündungen der Kanäle in die Gelenkfläche;
Fig. 2 eine perspektivische Ansicht einer weiteren Ausführungsform der erfindungsgemässen Gelenkendoprothese;
Fig. 3 einen Schnitt parallel zur ersten Drehachse durch die in Fig. 2 dargestellte Ausführungsform;
Fig. 4 einen Schnitt parallel zur zweiten Drehachse durch die in den Fig. 2 und 3 dargestellte Ausführungsform; und
Fig. 5 eine Aufsicht auf die in den Fig. 2 bis 4 dargestellte Ausführungsform.

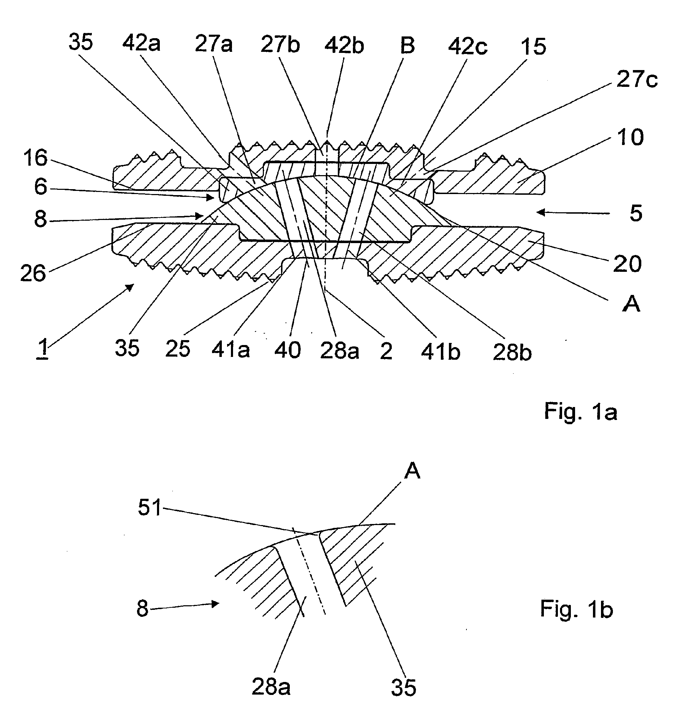

In Fig. 1 ist eine Ausführungsform der Gelenkprothese als Zwischenwirbelimplantat 1 in einem medio-lateralen Schnitt dargestellt. Das Zwischenwirbelimplantat 1 umfasst bezüglich seiner zur Wirbelsäulenlängsachse im wesentlichen parallelen Zentralachse 2 axial übereinander angeordnet ein oberes Teil 10 und ein unteres Teil 20, welche ein Gelenk 5 einschliessen. Das obere Teil 10 hat axial aussenstehend eine die Zentralachse 2 schneidende obere Appositionsfläche 15 zur Anlage an die Grundplatte eines oberhalb angrenzenden Wirbelkörpers und innen eine untere Oberfläche 16 mit dem integrierten, oberen Gelenkteil 6. Analog dazu hat das untere Teil 20 axial aussenstehend eine die Zentralachse 2 schneidende untere Appositionsfläche 25 zur Anlage an die Deckplatte eines unterhalb angrenzenden Wirbelkörpers und innen eine obere Oberfläche 16 mit dem integrierten unteren Gelenkteil 8. Das untere Gelenkteil 8 ist hier als Kugelsegment 35 ausgestaltet, während das obere Gelenkteil 6 als zum Kugelsegment 35 komplementäre Gelenkschale 36 ausgebildet ist, so dass die zwei Teile 10;20 durch das Gelenk 5 relativ zueinander polyaxial schwenkbar sind. Jedes der zwei Gelenkteile 6;8 ist als separates Teil ausgebildet und mit dem entsprechenden Teil 10;20 verbunden.

Am oberen Teil 10 sind obere Kanäle 27 angebracht, welche in die obere Appositionsfläche 15 münden und das obere Teil 10 sowie das obere Gelenkteil 6 bis zur Gelenkfläche B der Gelenkschale 36 durchdringen. Durch diese oberen Kanäle 27 wird die Schmiereigenschaften aufweisende Körperflüssigkeit von oben an die Gelenkflächen A;B des Gelenkes 5 transportiert. Analog sind am unteren Teil 20 untere Kanäle 28 angeordnet, welche in die untere Appositionsfläche 25 münden und das untere Teil 20 sowie das untere Gelenkteil 8 bis zur Gelenkfläche A des Kugelsegmentes 35 durchdringen, so dass die Körperflüssigkeit von unten an die Gelenkflächen A;B des Gelenkes 5 transportiert werden kann. Die Kanäle 27;28 sind in beiden Teilen 10;20 so angeordnet, dass ihre Öffnungen 40 in den Appositionsflächen 15;25 zentral liegen. In der hier dargestellten Ausführungsform des Zwischenwirbelimplantates 1 ist das obere Gelenkteil 6 mit drei oberen Kanälen 27a-c ausgebildet , wobei die Längsachse 42b des zentralen oberen Kanales 27b in der Ausgangslage des Zwischenwirbelimplantates 1 koaxial zur Längsachse 2 verläuft und die Längsachsen 42a;42c der zwei seitlichen oberen Kanäle 27a;27c mit der Zentralachse 2 einen Winkel einschliessen, wobei hier die Längsachse 42a des Kanals 27a mit der Zentralachse 2 einen Winkel von -50° einschliesst und die Längsachse 42c des Kanals 27c mit der Zentralachse 2 einen Winkel von +50° einschliesst. Das untere Gelenkteil 8 umfasst zwei untere Kanäle 28a;28b, deren Längsachsen 41 a;42b ebenfalls mit der Zentralachse 2 einen Winkel einschliessen, wobei die Längsachse 41 a des Kanals 28a mit der Zentralachse 2 einen Winkel von -15° einschliesst und die Längsachse 41 b des Kanals 28b mit der Zentralachse 2 einen Winkel von +15° einschliesst.

In Fig. 1b ist ein Ausschnitt aus dem unteren Gelenkteil 8 mit einem unteren Kanal 28a dargestellt. Der untere Kanal 28a durchdringt das Kugelsegment 35 bis an die Gelenkfläche A, wobei die Kanalwand am Übergang mit einer Rundung 51 versehen ist, so dass die Mündung des Kanals 28a in die Gelenkfläche A nicht scharfkantig sonder abgerundet ausgebildet ist. Die Mündungen des anderen unteren Kanals 28b sowie aller oberen Kanäle 27a-c sind vorzugsweise analog ausgestaltet.

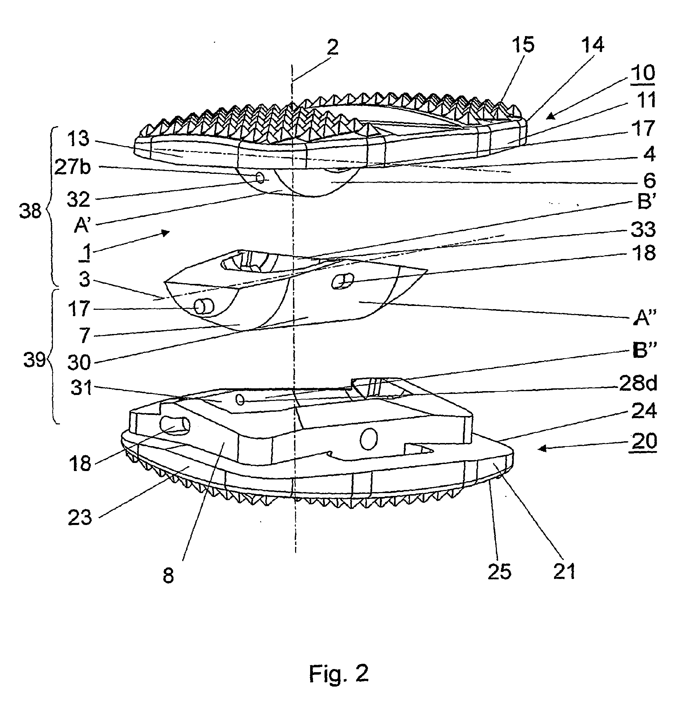

In den Fig. 2 bis 5 ist eine weitere als Zwischenwirbelimplantat 1 ausgebildete Ausführungsform der Gelenkendoprothese dargestellt. Das Zwischenwirbelimplantat 1 umfasst ein oberes Teil 10 mit einer oberen, quer zur Zentralachse 2 angeordneten Appositionsfläche 15 zur Anlage an die Grundplatte eines angrenzenden Wirbelkörpers, ein unteres Teil 20 mit einer unteren, quer zur Zentralachse 2 angeordneten Appositionsfläche 25 zur Anlage an die Deckplatte des angrenzenden Wirbelkörpers und dazwischen liegend ein Gelenk 5. Durch dieses Gelenk 5 sind das obere Teil 10 und das untere Teil 20 um zwei senkrecht zueinander stehende Drehachsen 3;4 relativ zueinander bewegbar verbunden.

Das Gelenk 5 ist durch drei Gelenkteile 6;7;8 realisiert, wovon das untere Gelenkteil 8 und das obere Gelenkteil 6 je ein mit dem mittleren Gelenkteil 7 zusammenwirkendes, um je eine Drehachse 3;4 rotierbares Drehgelenk 38;39 bilden. Das untere Drehgelenk 39 umfasst als Gelenkflächen eine am mittleren Gelenkteil 7 angeordnete zur ersten Drehachse 3 koaxiale, untere konvexe Gelenkfläche 30 und eine am unteren Gelenkteil 8 angeordnete, zur Gelenkfläche 30 komplementäre, untere konkave Gelenkfläche 31. Das obere Drehgelenk 38 umfasst als Gelenkflächen eine am oberen Gelenkteil 6 angeordnete zur zweiten Drehachse 4 koaxiale, obere konvexe Gelenkfläche 32 und eine am mittleren Gelenkteil 7 angeordnete, zur Gelenkfläche 32 komplementäre, obere konkave Gelenkfläche 33. Die Gelenkflächen 30;31;32;33 sind als Teilflächen von Kreiszylindermantelflächen mit axial endständig angrenzenden Kegelmantelteilflächen ausgestaltet.

Ferner sind am oberen und am mittleren Gelenkteil 6;7 axial endständig zu den Drehachsen 3;4 koaxiale Nocken 17 angebracht, welche in Langlochführungen 18 im unteren Gelenkteil 8 und im mittleren Gelenkteil 7 verschiebbar aufgenommen sind. Durch die in den Langlochführungen 18 geführten Nocken 18 werden die Drehwinkel der Gelenkteile 6;7;8 um die Drehachsen 3;4 begrenzt. Zudem wird das Zwischenwirbelimplantat 1 durch die in den Langlochführungen 18 aufgenommenen Nocken 17 zusammengehalten.

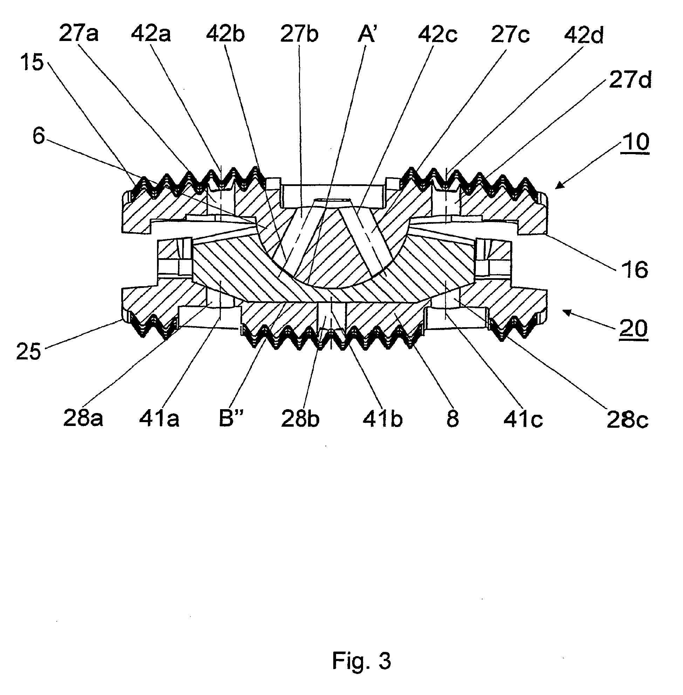

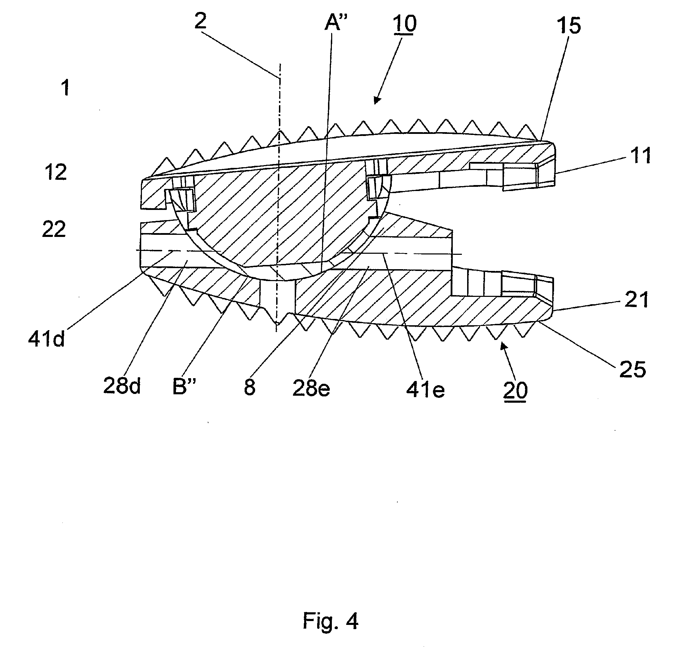

Wie in den Fig. 3 und 4 gezeigt sind am oberen Teil 10 vier obere Kanäle 27a-d angebracht, welche in die obere Appositionsfläche 15 münden und das obere Teil 10 einschliesslich des oberen Gelenkteiles 6 bis zur Gelenkfläche A' durchdringen. Durch diese Kanäle 27 wird die Schmiereigenschaften aufweisende Körperflüssigkeit von oben an die Gelenkflächen A';B' des oberen Drehgelenkes 38 (Fig. 2) transportiert. Am unteren Teil 20 sind drei untere Kanäle 28a-c angeordnet, welche in die untere Appositionsfläche 25 münden und das untere Teil 20 einschliesslich des unteren unten an die Gelenkflächen A";B" des unteren Drehgelenkes 39 (Fig. 2) transportiert werden kann. Ferner sind am unteren Gelenkteil 8 zwei weitere untere Kanäle 28d;28e angebracht (Fig. 4), deren Längsachsen 41d;41e senkrecht zur Zentralachse 2 stehen und welche aussen von der ventralen und der dorsalen Seitenfläche 21 ;22 her in das unter Gelenkteil 8 eindringen und das untere Teil 20 einschliesslich des unteren Gelenkteiles 8 bis zu den Gelenkflächen A";B" durchdringen. Wie aus dem in Fig. 3 dargestellten medio-lateralen Schnitt ersichtlich münden nur zwei der vier oberen Kanäle 27b;27c in die Gelenkfläche A' des oberen Gelenkteiles 6. Die anderen beiden oberen Kanäle 27a;27d münden in die untere Oberfläche 16 des oberen Teiles 10 und haben Längsachsen 42a;42d, welche parallel zur Zentralachse 2 gerichtet sind. Die Längsachsen 42b;42c der zwei oberen Kanäle 27b;27c schliessen mit der Zentralachse 2 einen Winkel ein, wobei die Längsachse 42b des oberen Kanals 27b mit der Zentralachse 2 einen Winkel von +25° einschliesst und die Längsachse 42c des Kanals 27c mit der Zentralachse 2 einen Winkel von -25° einschliesst. Ferner sind aus dem in Fig. 4 dargestellten antero-posterioren Schnitt ersichtlich, dass die zwei unteren Kanäle 28d;28e quer zur Zentralachse 2 stehende Längsachsen 41d;41e aufweisen, wobei die Längsachse 41 d des unteren Kanals 28d einen Winkel von +90° mit der Zentralachse 2 einschliesst während die Längsachse 41 e des unteren Kanals 28e einen Winkel von - 90° mit der Zentralachse 2 einschliesst. Beide Längsachsen 41d;41e liegen in der antero-posterioren Mittelebene des Zwischenwirbelimplantates 1.

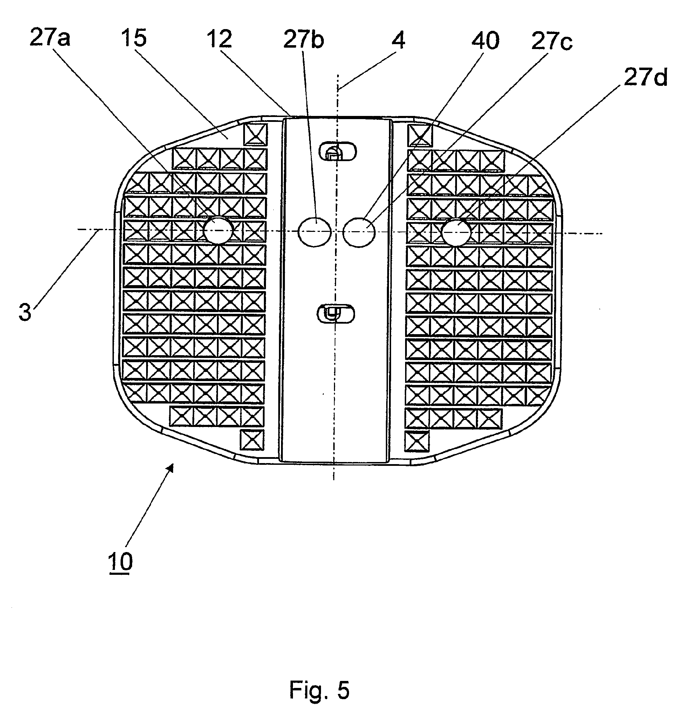

Fig. 5 zeigt die Öffnungen 40 der in die obere Appositionsfläche 15 mündenden oberen Kanäle 27a-d. Die Öffnungen 40 liegen in einer Teilfläche 37 der Appositionsfläche 15, welche orthogonal zur Zentralachse 2 steht und zu dieser konzentrisch ist. Die Zentralachse 2 (Fig. 2) schneidet den Schnittpunkt der ersten mit der zweiten Drehachse 3;4 und ist gegen die dorsale Seitenfläche 12 des oberen Teiles 10 aus dessen Mitte verschoben.

## Patentansprüche

1. Gelenkendoprothese mit einer Zentralachse (2), einem oberen Teil (10), einem unteren Teil (20) und einem dazwischen angeordneten Gelenk (5), wobei
A) das Gelenk (5) mindestens zwei aufeinander gleitbare Gelenkflächen A und B umfasst;
B) das obere Teil (10) eine die Zentralachse (2) schneidende, obere Appositionsfläche (15) und Seitenflächen (11;12;13;14) aufweist;
C) das untere Teil (20) eine die Zentralachse (2) schneidende, untere Appositionsfläche (25) und Seitenflächen (21;22;23;24) aufweist, wobei
D) die Gelenkendoprothese mindestens einen für den Transport von Körperflüssigkeit geeigneten Kanal (27;28) umfasst, der in eine der beiden Gelenkflächen A und B mündet und diese mit dem Äusseren der Gelenkendoprothese verbindet, und
E) das Gelenk (5) ein oberes, ein mittleres und ein unteres Gelenkteil (6;7;8) umfasst,
**dadurch gekennzeichnet, dass**
F) die Gelenkendoprothese in Form eines Zwischenwirbelimplantats (1) realisiert ist und die beiden Appositionsflächen (15,25) zur Anlage an die Grundplatten der betroffenen Wirbelkörper geeignet sind.

2. Gelenkendoprothese nach Anspruch 1, **dadurch gekennzeichnet, dass** mindestens ein oberer Kanal (27) in die obere Appositionsfläche (15) mündet.

3. Gelenkendoprothese nach Anspruch 1, **dadurch gekennzeichnet, dass** mindestens ein unterer Kanal (28) in die untere Appositionsfläche (25) mündet.

4. Gelenkendoprothese nach Anspruch 1, dass mindestens ein oberer Kanal (27) in die obere Appositionsfläche (15) mündet und mindestens ein unterer Kanal (28) in die untere Appositionsfläche (25) mündet.

5. Gelenkendoprothese nach Anspruch 4, **dadurch gekennzeichnet, dass** die Mündungen der Kanäle (27;28) in die Gelenkflächen A;B mit Rundungen (51) versehen sind.

6. Gelenkendoprothese nach einem der Ansprüche 2, 4 oder 5, **dadurch gekennzeichnet, dass** sie mehrere obere Kanäle (27) umfasst.

7. Gelenkendoprothese nach einem der Ansprüche 3 bis 5, **dadurch gekennzeichnet, dass** sie mehrere untere Kanäle (28) umfasst.

8. Gelenkendoprothese nach einem der Ansprüche 4 - 7, **dadurch gekennzeichnet, dass** sie mehrere obere Kanäle (27) und mehrere untere Kanäle (28) umfasst.

9. Gelenkendoprothese nach Anspruch 8, **dadurch gekennzeichnet, dass** die oberen und die unteren Kanäle (27;28) Längsachsen (42;41) aufweisen und derart angeordnet sind, dass die Längsachsen (42;41) mit der Zentralachse (2) einen Winkel zwischen 0° und 90° einschliessen.

10. Gelenkendoprothese nach einem der Ansprüche 4 bis 9, **dadurch gekennzeichnet, dass** jeder Kanal (27;28) eine Querschnittsfläche aufweist, welche zwischen 0,01 % und 10% einer Gelenkfläche A;B beträgt.

11. Gelenkendoprothese nach einem der Ansprüche 4 bis 10, **dadurch gekennzeichnet, dass** die Kanäle (27;28) einen Durchmesser zwischen 0,3 mm und 4,0 mm aufweisen.

12. Gelenkendoprothese nach einem der Ansprüche 4 bis 11, **dadurch gekennzeichnet, dass** die Kanäle (27;28) symmetrisch verteilt in die Gelenkflächen A;B münden.

13. Gelenkendoprothese nach einem der Ansprüche 6 bis 12, **dadurch gekennzeichnet, dass** die obere Appositionsfläche (15) einen Flächeninhalt Fₒ aufweist und die oberen Kanäle (27) innerhalb einer Teilfläche (37) mit einem Flächeninhalt Tₒ < Fₒ in die obere Appositionsfläche (15) münden.

14. Gelenkendoprothese nach Anspruch 13, **dadurch gekennzeichnet, dass** der Flächeninhalt Tₒ der Teilfläche zwischen 0,006 % und 1,0 % des Flächeninhaltes Fₒ der oberen Appositionsfläche (15) beträgt.

15. Gelenkendoprothese nach einem der Ansprüche 7 bis 14, **dadurch gekennzeichnet, dass** die untere Appositionsfläche (25) einen Flächeninhalt Fᵤ aufweist und die unteren Kanäle (28) innerhalb einer Teilfläche (37) mit einem Flächeninhalt Tᵤ < Fᵤ in die untere Appositionsfläche (25) münden.

16. Gelenkendoprothese nach Anspruch 15, **dadurch gekennzeichnet, dass** der Flächeninhalt Tᵤ der Teilfläche zwischen 0,006 % und 1,0 % des Flächeninhaltes Fᵤ der unteren Appositionsfläche (25) beträgt.

17. Gelenkendoprothese nach einem der Ansprüche 13 bis 16, **dadurch gekennzeichnet, dass** die Teilflächen konzentrisch zur Zentralachse (2) angeordnet sind.

18. Gelenkendoprothese nach einem der Ansprüche 9 bis 17, **dadurch gekennzeichnet, dass** die Längsachsen der oberen Kanäle (27) mit der Zentralachse (2) einen Winkel zwischen 0° und 65° einschliessen.

19. Gelenkendoprothese nach einem der Ansprüche 1 bis 18, **dadurch gekennzeichnet, dass** mindestens die Gelenkflächen A;B der das Gelenk (5) bildenden Gelenkteile (6;7;8) aus der Materialpaarung Metall/Polymer, Metall/Metall oder Keramik/Keramik hergestellt sind.

## Claims

1. An endoprosthesis for a joint, with a central axis (2), a top part (10), a bottom part (20) and a joint (5) provided between them, wherein
A) the joint (5) comprises at least two articular surfaces A and B that can slide on one another;
B) the top part (10) has a top apposed surface (15) that intersects the central axis (2) and lateral surfaces (11, 12, 13, 14);
C) the bottom part (20) has a bottom apposed surface (25) that intersects the central axis (2) and lateral surfaces (21, 22, 23, 24); whereby
D) the endoprosthesis for a joint comprises at least one channel (27, 28) suitable to convey body fluid, said channel terminating in one of the two articular surfaces A and B and connecting it with the exterior of the endoprosthesis for the joint, and
E) the joint (5) comprises a top, a central and a bottom joint part (6;7;8),
**characterised in that**
F) the endoprosthesis is realised in the form of an intervertebral implant (1) and that the two apposed surfaces (15;25) are suitable for contact with the end plates of the respective intervertebral bodies.

2. An endoprosthesis for a joint according to claim 1, **characterised in that** at least one upper channel (27) terminates in the top apposed surface (15).

3. An endoprosthesis for a joint according to claim 1, **characterised in that** at least one lower channel (28) terminates in the bottom apposed surface (25).

4. An endoprosthesis for a joint according to claim 1, **characterised in that** at least one upper channel (27) terminates in the top apposed surface (15) and at least one lower channel (28) terminates in the bottom apposed surface (25).

5. An endoprosthesis for a joint according to claim 4, **characterised in that** the terminations of the channels (27, 28) are rounded (51) in the articular surfaces A, B.

6. An endoprosthesis for a joint according to any one of claims 2, 4 or 5, **characterised in that** it has a plurality of upper channels (27).

7. An endoprosthesis for a joint according to any one of claims 3 to 5, **characterised in that** it has a plurality of lower channels (28).

8. An endoprosthesis for a joint according to any one of claims 4 to 7, **characterised in that** it has a plurality of upper channels (27) and a plurality of lower channels (28).

9. An endoprosthesis for a joint according to claim 8, **characterised in that** the upper and lower channels (27, 28) have longitudinal axes 42, 41 and are so arranged, that the longitudinal axes 42, 41 include an angle between 0° and 90° with the central axis (2).

10. An endoprosthesis for a joint according to any one of claims 4 to 9, **characterised in that** each channel (27, 28) has a cross-section that is between 0.01% and 10% of the articular surface A, B.

11. An endoprosthesis for a joint according to any one of claims 4 to 10, **characterised in that** the channels (27, 28) have a diameter between 0.3 mm and 4.0 mm.

12. An endoprosthesis for a joint according to any one of claims 4 to 11, **characterised in that** the channels (27, 28) terminate symmetrically distributed in the articular surfaces A, B.

13. An endoprosthesis for a joint according to any one of claims 6 to 12, **characterised in that** the top apposed surface (15) has a surface area of Fₒ and the upper channels (27) terminate in the top apposed surface (15) within a part surface (37) with a surface area of Tₒ<Fₒ.

14. An endoprosthesis for a joint according to claim 13, **characterised in that** the surface area Tₒ of the part surface is between 0.006% and 1.0% of the surface area Fₒ of the top apposed surface (15).

15. An endoprosthesis for a joint according to any one of claims 7 to 14, **characterised in that** the bottom apposed surface (25) has a surface area of Fᵤ and the lower channels (28) terminate in the bottom apposed surface (25) within a part surface (37) with a surface area of Tᵤ<Fᵤ.

16. An endoprosthesis for a joint according to claim 15, **characterised in that** the surface area Tᵤ of the part surface is between 0.006% and 1.0% of the surface area Fᵤ of the bottom apposed surface (25).

17. An endoprosthesis for a joint according to any one of claims 13 to 16, **characterised in that** the part surfaces are arranged concentrically with the central axis (2).

18. An endoprosthesis for a joint according to any one of claims 9 to 17, **characterised in that** the longitudinal axes of the upper channels (27) include an angle between 0° and 65° with the central axis (2).

19. An endoprosthesis for a joint according to any one of the claims 1 to 18, **characterised in that** at least the articular surfaces A, B of the joint parts (6, 7, 8) forming the joint (5) are produced from material pairs of metal/polymer, metal/metal or ceramics/ceramics.

## Revendications

1. Endoprothèse d'articulation comprenant un axe central (2), une partie supérieure (10), une partie inférieure (20) et une articulation (5) disposée entre celles-ci, dans laquelle
A) l'articulation (5) comprend au moins deux surfaces d'articulation A et B qui peuvent glisser l'une sur l'autre ;
B) la partie supérieure (10) présente une surface d'apposition supérieure (15) coupant l'axe central (2) et des faces latérales (11 ; 12 ; 13 ; 14) ;
C) la partie inférieure (20) présente une surface d'apposition inférieure (25) coupant l'axe central (2) et des faces latérales (21 ; 22 ; 23 ; 24),
D) l'endoprothèse d'articulation comprenant au moins un canal approprié (27 ; 28) pour le transport de fluide corporel qui débouche dans une des deux surfaces d'articulation A et B et relie celles-ci à la partie externe de l'endoprothèse d'articulation, et
E) l'articulation (5) comprend une partie d'articulation supérieure, intermédiaire et inférieure (6 ; 7 ; 8);
**caractérisée en ce que**
F) l'endoprothèse d'articulation est réalisée en forme d'un implant intervertébral (1) et les deux surfaces d'apposition (15,25) sont appropriées à s'appuyer contre les plateaux inférieurs des corps vertébraux concernés.

2. Endoprothèse d'articulation selon la revendication 1, **caractérisée en ce qu'**au moins un canal supérieur (27) débouche dans la surface d'apposition supérieure (15).

3. Endoprothèse d'articulation selon la revendication 1, **caractérisée en ce qu'**au moins un canal inférieur (28) débouche dans la surface d'apposition inférieure (25).

4. Endoprothèse d'articulation selon la revendication 1, **caractérisée en ce qu'**au moins un canal supérieur (27) débouche dans la surface d'apposition supérieure (15) et au moins un canal inférieur (28) débouche dans la surface d'apposition inférieure (25).

5. Endoprothèse d'articulation selon la revendication 4, **caractérisée en ce que** les embouchures des canaux (27 ; 28) dans les surfaces d'articulation A ; B sont pourvues de parties arrondies (51).

6. Endoprothèse d'articulation selon l'une quelconque des revendications 2, 4 ou 5, **caractérisée en ce qu'**elle comprend plusieurs canaux supérieurs (27).

7. Endoprothèse d'articulation selon l'une quelconque des revendications 3 à 5, **caractérisée en ce qu'**elle comprend plusieurs canaux inférieurs (28).

8. Endoprothèse d'articulation selon l'une quelconque des revendications 4 à 7, **caractérisée en ce qu'**elle comprend plusieurs canaux supérieurs (27) et plusieurs canaux inférieurs (28).

9. Endoprothèse d'articulation selon la revendication 8, **caractérisée en ce que** les canaux supérieurs et inférieurs (27 ; 28) présentent des axes longitudinaux (42 ; 41) et sont disposés de telle façon que les axes longitudinaux (42 ; 41) forment un angle compris entre 0° et 90° avec l'axe central (2).

10. Endoprothèse d'articulation selon l'une quelconque des revendications 4 à 9, **caractérisée en ce que** chaque canal (27 ; 28) présente une aire de section transversale, laquelle varie entre 0,01% et 10% d'une surface d'articulation A ; B.

11. Endoprothèse d'articulation selon l'une quelconque des revendications 4 à 10, **caractérisée en ce que** les canaux (27 ; 28) présentent un diamètre compris entre 0,3 mm et 4,0 mm.

12. Endoprothèse d'articulation selon l'une quelconque des revendications 4 à 11, **caractérisée en ce que** les canaux (27 ; 28) débouchent de façon symétrique dans les surfaces d'articulation A ; B.

13. Endoprothèse d'articulation selon l'une quelconque des revendications 6 à 12, **caractérisée en ce que** la surface d'apposition supérieure (15) présente une aire Fₒ et les canaux supérieurs (27) débouchent dans la surface d'apposition supérieure (15) au niveau d'une surface partielle (37) ayant une aire Tₒ < Fₒ.

14. Endoprothèse d'articulation selon la revendication 13, **caractérisée en ce que** l'aire Tₒ de la surface partielle varie entre 0,006% et 1,0% de l'aire Fₒ de la surface d'apposition supérieure (15).

15. Endoprothèse d'articulation selon l'une quelconque des revendications 7 à 14, **caractérisée en ce que** la surface d'apposition inférieure (25) présente une aire Fᵤ et les canaux inférieurs (28) débouchent dans la surface d'apposition inférieure (25) au niveau d'une surface partielle (37) ayant une aire Tᵤ < Fᵤ.

16. Endoprothèse d'articulation selon la revendication 15, **caractérisée en ce que** l'aire Tᵤ de la surface partielle varie entre 0,006% et 1,0% de l'aire Fᵤ de la surface d'apposition inférieure (25).

17. Endoprothèse d'articulation selon l'une quelconque des revendications 13 à 16, **caractérisée en ce que** les surfaces partielles sont disposées concentriquement par rapport à l'axe central (2).

18. Endoprothèse d'articulation selon l'une quelconque des revendications 9 à 17, **caractérisée en ce que** les axes longitudinaux des canaux supérieurs (27) forment un angle compris entre 0° et 65° avec l'axe central (2).

19. Endoprothèse d'articulation selon l'une quelconque des revendications 1 à 18, **caractérisée en ce qu'**au moins les surfaces d'articulation A ; B des parties d'articulation (6 ; 7 ; 8) formant l'articulation (5) sont fabriquées à partir d'un matériau composite métal/polymère, métal/métal ou céramique/céramique.
